# EUROPEAN PATENT APPLICATION

(11) **EP 2 596 830 A1**
(43) Date of publication of application: **29.05.2013**
(21) Application number: 12187983.7
(22) Date of filing: 10.10.2012
(51) Int. Cl.: A61M 25/09

(54) **Guidewire**

(30) Priority: 22.11.2011 JP 2011255490
(71) Applicant: ASAHI INTECC CO., LTD., Nagoya-shi, Aichi 463-0024 (JP)
(72) Inventor: Miyata, Naohiko, Nagoya-shi, Aichi 463-0024 (JP); Takemoto, Hirokatsu, Nagoya-shi, Aichi 463-0024 (JP)
(74) Representative: Winter, Brandl, Fürniss, Hübner, Röss, Kaiser, Polte - Partnerschaft

(57) **Abstract**

A guidewire (1A, 1B, 1C, 1D, 1E, 1F) includes a core wire (2), a coil body (3) that covers the core wire (2), a tip portion (4) that fixes a distal end of the core wire (2) and a distal end of the coil body (3) to each other, and a resin layer (6) formed on an outer peripheral surface of the core wire (2). A gap is provided between an outer peripheral surface of the resin layer (6) and an inner peripheral surface of the coil body (3) so that the resin layer (6) and the coil body (3) are not in contact with each other.

## Description

### BACKGROUND OF THE INVENTION

### 1. Field of the Invention

The present invention relates to a guidewire suitable for use in the medical field.

### 2. Description of the Related Art

Medical guidewires used as guides for inserting a catheter into, for example, a blood vessel, a ureter, or an organ, or inserting an indwelling device into an aneurysm of a blood vessel are well known in the medical field.

A guidewire including a tapered coil body provided with resin portions on the inner and outer sides thereof is also known (see, for example, Japanese Unexamined Patent Application Publication No. 2010-268888). In addition, a guidewire having a double-coil-body structure which includes a core wire fixed to a plurality of coil bodies by a fixing portion and in which the coil bodies exist on both the distal-end side and proximal-end side of the fixing portion is also known (see, for example, United States Patent No. 5,345,945).

### SUMMARY OF THE INVENTION

However, according to Japanese Unexamined Patent Application Publication No. 2010-268888, the inner space of a coil spring that is provided in a distal end portion of the guidewire is filled with a resin portion, and the resin portion is connected to a tip portion of the guidewire and the coil spring. Therefore, although the torque transmission performance of the distal end portion of the guidewire and supportability that affects the insertability of a medical device, such as a balloon catheter or a stent, may be increased, there is a problem that the flexural rigidity of the distal end portion is excessively high and sufficient flexibility cannot be obtained. When the flexibility of the distal end portion is low, insertability of the guidewire into a peripheral portion, such as a peripheral blood vessel, will be reduced.

The guidewire disclosed in United States Patent No. 5,345,945 has the double-coil-body structure. Therefore, the rigidity of a portion of the guidewire that is closer to the distal end thereof than the fixing portion which fixes the core wire to the coil bodies is increased, and the torque transmission performance and supportability are increased accordingly. However, there is a problem that the rigidity of a portion of the guidewire that is closer to the proximal end thereof than the fixing portion cannot be increased by the double-coil-body structure and therefore sufficient supportability cannot be obtained.

The present invention has been made in view of the above-described problems, and an object of the present invention is to provide a guidewire in which supportability of a distal end portion of the guidewire is increased and good flexibility is ensured so that insertability of the guidewire into a peripheral portion is increased.

According to an aspect of the present invention, the guidewire includes a core wire, a coil body that covers at least a distal end portion of the core wire, a tip portion that fixes a distal end of the core wire and a distal end of the coil body to each other, and a resin layer formed at least inside the coil body and on an outer peripheral surface of the core wire. An outer peripheral surface of the resin layer and an inner peripheral surface of the coil body face each other with a gap therebetween so that the resin layer and the coil body are not in contact with each other.

In this structure, since the resin layer is formed at least on the outer peripheral surface of the distal end portion of the core wire, the distal end portion of the guidewire has sufficient supportability. In addition, since the resin layer is not in contact with the coil body that surrounds the resin layer, the resin layer and the coil body may be deformed into, for example, a curved or bent shape without interfering with each other. Therefore, the resin layer and the coil body can be appropriately and sufficiently deformed into a curved or bent shape when the distal end portion of the guidewire is deformed into a curved or bent shape. Thus, the distal end portion of the guidewire has sufficient flexibility and insertability of the guidewire into a peripheral portion can be increased.

According to the guidewire of the present invention, supportability of the distal end portion is increased and good flexibility is ensured so that insertability of the guidewire into a peripheral portion is also increased.

### BRIEF DESCRIPTION OF THE DRAWINGS

Fig. 1 illustrates a guidewire according to a first embodiment.
Fig. 2A is a vertical sectional view of a cylindrical body made of resin.
Fig. 2B is a sectional view of Fig. 2A taken along line IIB-IIB.
Fig. 3 illustrates a guidewire according to a second embodiment.
Fig. 4 illustrates a guidewire according to a third embodiment.
Fig. 5 illustrates a guidewire according to a fourth embodiment.
Fig. 6 illustrates a guidewire according to a fifth embodiment.
Fig. 7 illustrates a guidewire according to a sixth embodiment.

### DESCRIPTION OF THE PREFERRED EMBODIMENTS

Guidewires according to first to sixth embodiments of the present invention will now be described.

### First Embodiment

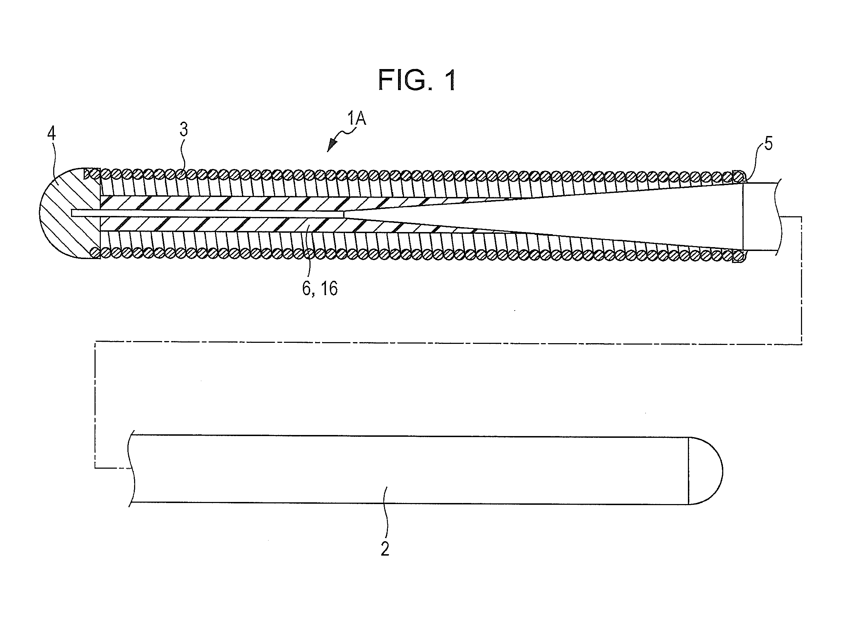

Referring to Fig. 1, a guidewire 1A, which is used for a medical purpose, includes a round-bar-shaped core wire 2 made of a metal material. The core wire 2 is tapered such that the diameter thereof is small at the distal end and large at the proximal end.

A wound coil body 3 made of a metal material is arranged so as to cover a distal end portion of the core wire 2. A substantially hemispherical tip portion 4 is formed so as to fix the distal end of the core wire 2 and the distal end of the coil body 3 together. The proximal end of the coil body 3 is fixed to the core wire 2 by a first fixing portion 5.

A resin layer 6 is formed at least on the outer peripheral surface of the distal end portion of the core wire 2 and inside the coil body 3. The distal end of the resin layer 6 is in contact with the proximal end of the tip portion 4. The outer peripheral surface of the resin layer 6 and the inner peripheral surface of the coil body 3 face each other with a gap therebetween so that the resin layer 6 and the coil body 3 are not in contact with each other.

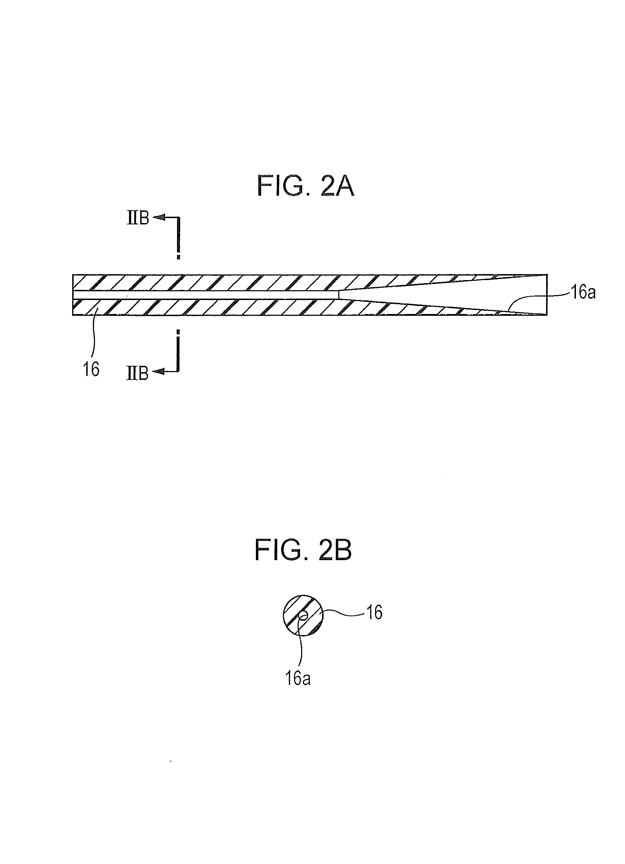

The resin layer 6 includes a cylindrical body 16 made of resin, as illustrated in Fig. 2, and is easily formed by inserting the core wire 2 into a cylinder hole 16a in the cylindrical body 16. The dimensions and shapes of the cylindrical body 16 and the cylinder hole 16a desirably correspond to the external shape of a portion of the core wire 2 on which the resin layer 6 is formed. Polyurethane, polyamide, polyimide, and various elastomers, for example, are suitable as the material of the cylindrical body 16. In particular, the cylindrical body 16 is preferably made of polyimide since the flexural rigidity and supportability can be increased.

The first fixing portion 5 may be formed by a known technology (for example, adhesion, soldering, or welding) using a known material.

In the guidewire 1A having the above-described structure, the resin layer 6 is formed on the outer peripheral surface of the core wire 2. Therefore, the distal end portion of the guidewire 1A has sufficient supportability. In addition, since the resin layer 6 is not in contact with the coil body 3 that surrounds the resin layer 6, when the guidewire 1A is deformed into a curved shape, the resin layer 6 and the coil body 3 may be deformed without interfering with each other. Therefore, the resin layer 6 and the coil body 3 can be appropriately and sufficiently deformed into a curved or bent shape when the distal end portion of the guidewire 1A is deformed into a curved or bent shape. Thus, the distal end portion of the guidewire 1A has sufficient flexibility and insertability of the guidewire 1A into a peripheral portion can be increased.

In addition, the resin layer 6 is formed on the outer peripheral surface of the core wire 2 so as to extend from the proximal end of the tip portion 4 toward the proximal end of the core wire 2, and the resin layer 6 and the tip portion 4 are formed continuously with each other. Therefore, supportability of a portion from the tip portion 4 of the guidewire 1A to the proximal end of the resin layer 6 is further increased without reducing the flexibility of the distal end portion of the guidewire 1A.

### Second Embodiment

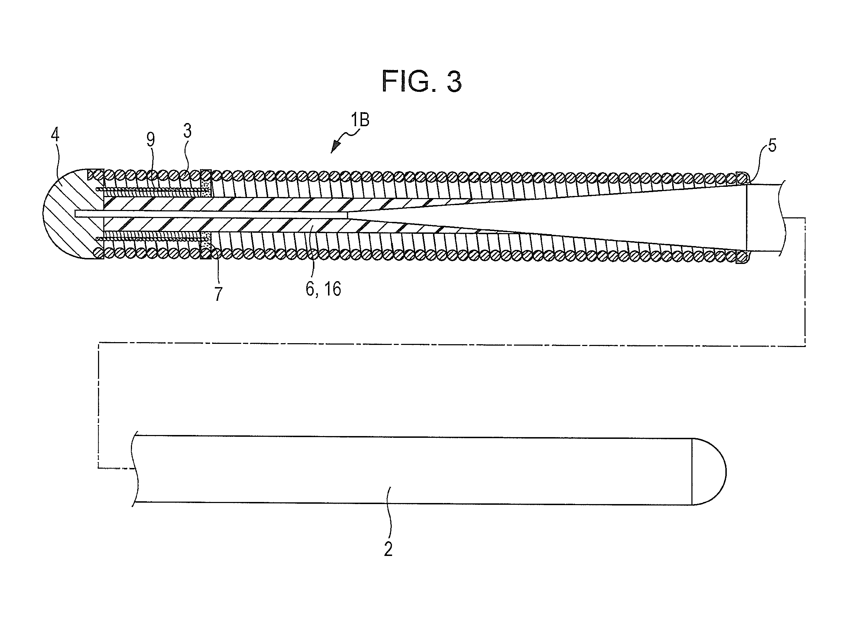

A guidewire 1B according to a second embodiment will now be described with reference to Fig. 3. Components similar to those of the first embodiment are denoted by the same reference numerals, and explanations thereof are thus omitted.

As illustrated in Fig. 3, the guidewire 1B includes an inner coil body 9 that is disposed inside the coil body 3 and covers the distal end portion of the core wire 2. More specifically, the distal end of the inner coil body 9 is fixed to the tip portion 4, and the proximal end of the inner coil body 9 is connected to a second fixing portion 7, which is separated from the tip portion 4 toward the proximal end of the guidewire 1A. The resin layer 6 is formed inside the inner coil body 9. The distal end of the resin layer 6 is in contact with the proximal end of the tip portion 4. The outer peripheral surface of the resin layer 6 and the inner peripheral surface of the inner coil body 9 face each other with a gap therebetween so that the resin layer 6 and the inner coil body 9 are not in contact with each other. The inner coil body 9 may be formed of a single-wire coil. However, from the viewpoint of increasing the supportability, the inner coil body 9 is preferably formed of a multiple-wire coil obtained by twisting a plurality of coil wires together.

When the inner coil body 9 is provided, the torque transmission performance and supportability can be increased. In addition, since the inner coil body 9 and the resin layer 6 are not in contact with each other, the flexibility of the distal end portion of the guidewire 1B is not reduced and good insertability of the guidewire 1B into a peripheral portion is ensured.

### Third Embodiment

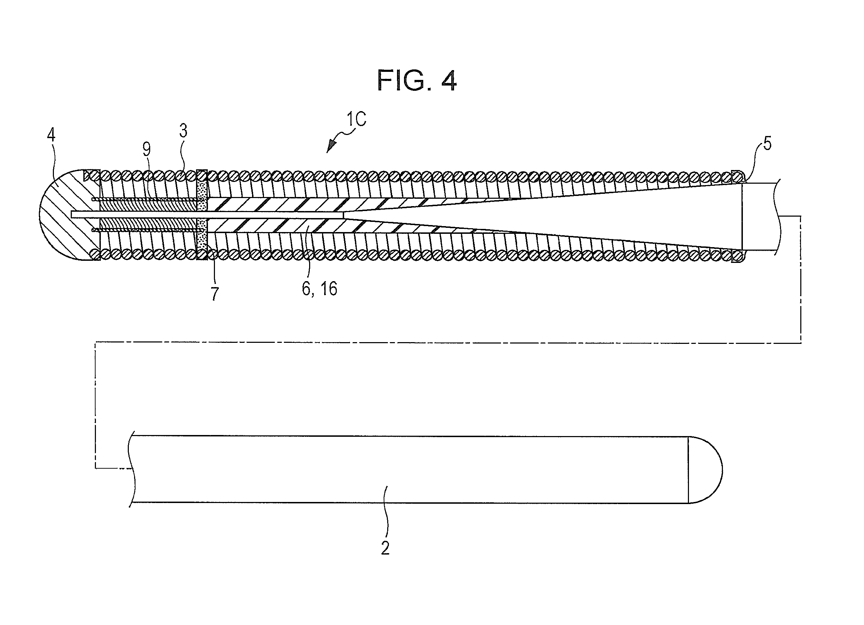

A guidewire 1C according to a third embodiment will now be described with reference to Fig. 4. Components similar to those of the first and second embodiments are denoted by the same reference numerals, and explanations thereof are thus omitted.

As illustrated in Fig. 4, the guidewire 1C includes the inner coil body 9, and the resin layer 6 is arranged on the proximal-end side of the inner coil body 9. More specifically, the distal end of the inner coil body 9 is fixed to the tip portion 4, and the proximal end of the inner coil body 9 is connected to the distal end of the resin layer 6 with the second fixing portion 7, which is formed at the distal end of the resin layer 6, interposed therebetween. The outer peripheral surface of the resin layer 6 is, of course, not in contact with the inner peripheral surface of the coil body 3.

In this structure, the resin layer 6 is separated from the tip portion 4 toward the proximal end of the guidewire 1C and the inner coil body 9 is disposed in the gap between the resin layer 6 and the tip portion 4. Therefore, the flexibility of a portion of the guidewire 1C between the distal end of the resin layer 6 and the tip portion 4 can be increased while good supportability of the distal end portion of the guidewire 1C is ensured. As a result, the insertability of the guidewire 1C into a peripheral portion can be increased.

### Fourth Embodiment

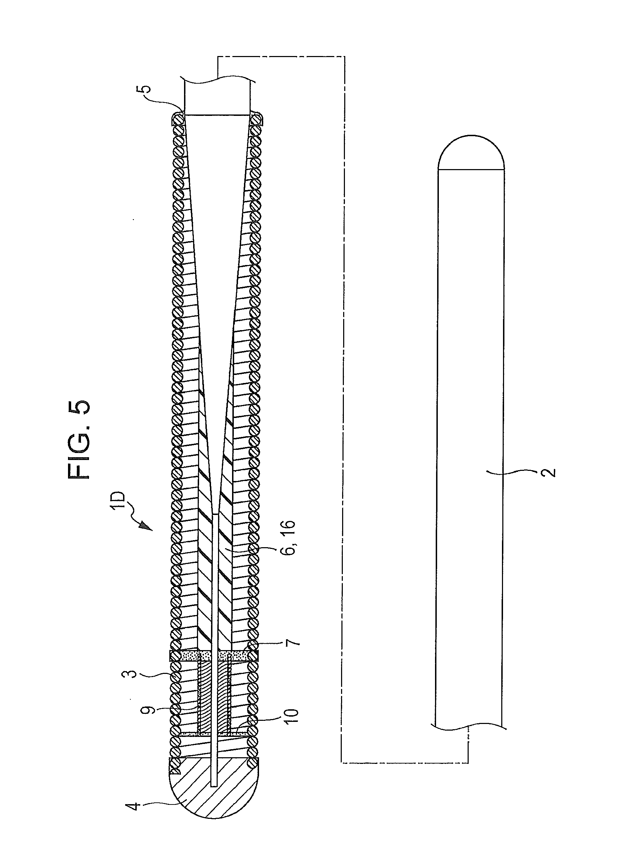

A guidewire 1D according to a fourth embodiment will now be described with reference to Fig. 5. Components similar to those of the first to third embodiments are denoted by the same reference numerals, and explanations thereof are thus omitted.

As illustrated in Fig. 5, the guidewire 1D includes the inner coil body 9, and the resin layer 6 is arranged on the proximal-end side of the inner coil body 9. The distal end of the inner coil body 9 is separated from the tip portion 4 toward the proximal end of the guidewire 1D. More specifically, the distal end of the inner coil body 9 is connected to a third fixing portion 10, which is separated from the tip portion 4 toward the proximal end of the guidewire 1D. The proximal end of the inner coil body 9 is connected to the distal end of the resin layer 6 with the second fixing portion 7 interposed therebetween.

In this structure, the distal end of the inner coil body 9 is separated from the tip portion 4 toward the proximal end of the guidewire 1D. Therefore, also in this case, the flexibility of a portion of the guidewire 1D between the tip portion 4 and the distal end of the inner coil body 9 can be increased while good supportability of the distal end portion of the guidewire 1D is ensured. As a result, the insertability of the guidewire 1D into a peripheral portion can be increased.

### Fifth Embodiment

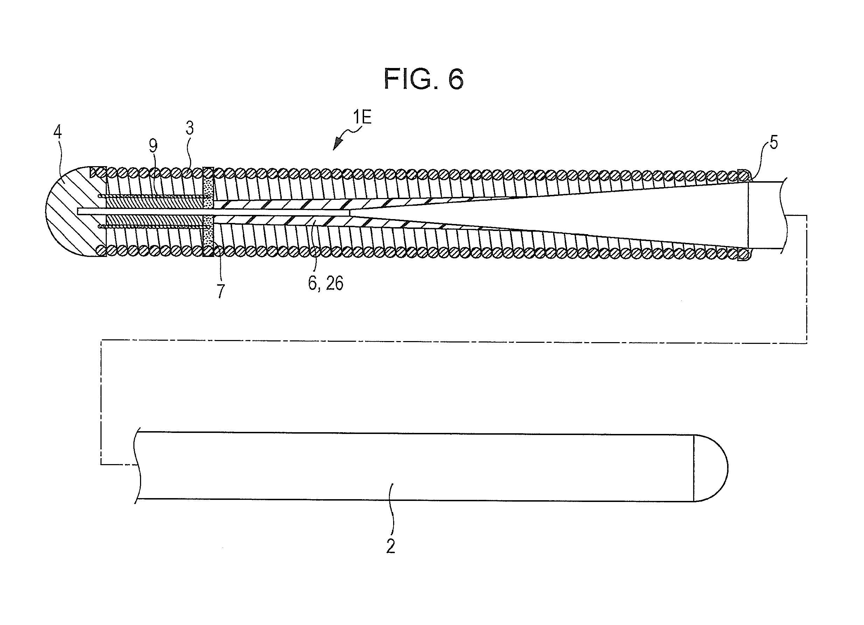

A guidewire 1E according to a fifth embodiment will now be described with reference to Fig. 6. Components similar to those of the first to fourth embodiments are denoted by the same reference numerals, and explanations thereof are thus omitted.

In the guidewire 1E illustrated in Fig. 6, the outer diameter of the resin layer 6 increases toward the proximal end of the core wire 2.

With this structure, supportability of the resin layer 6 increases toward the proximal end of the guidewire 1E. Accordingly, a medical device, such as a balloon catheter or a stent, can be smoothly inserted along the guidewire 1E. The resin layer 6 is preferably simply formed of a cylindrical body 26 made of resin which has a diameter that increases toward the proximal end of the guidewire 1E.

### Sixth Embodiment

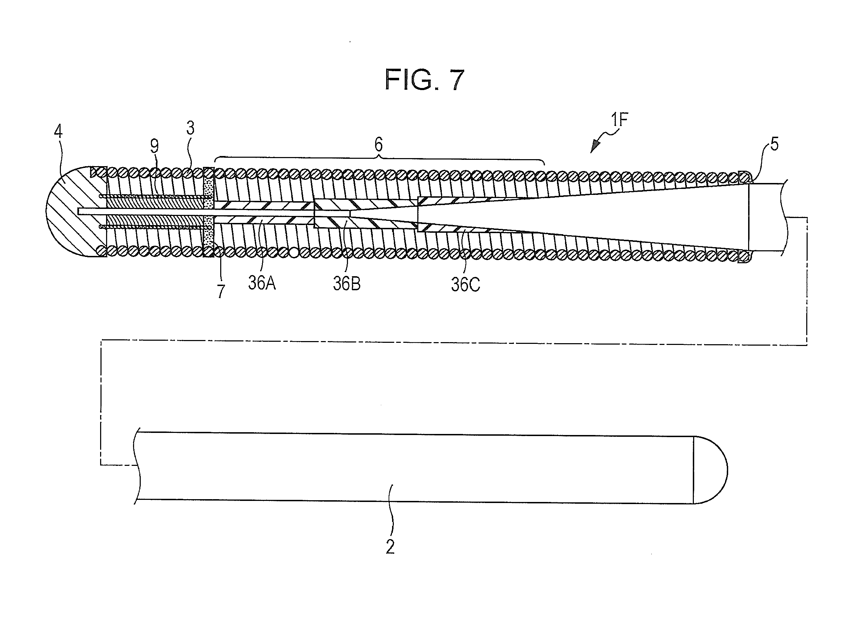

A guidewire 1F according to a sixth embodiment will now be described with reference to Fig. 7. Components similar to those of the first to fifth embodiments are denoted by the same reference numerals, and explanations thereof are thus omitted.

In the guidewire 1F illustrated in Fig. 7, the outer diameter of the resin layer 6 increases toward the proximal end of the core wire 2. The resin layer 6 includes three independent cylindrical bodies 36A to 36C made of resin that have different outer diameters. The resin layer 6 is formed by fitting the cylindrical bodies 36A to 36C onto the core wire 2 in descending order of outer diameter.

With this structure, the resin layer 6 having the rigidity that gradually changes can be easily formed simply by inserting the core wire 2 through the cylindrical bodies 36A to 36C. Although the resin layer 6 is formed of a plurality of cylindrical bodies 36A to 36C having different outer diameters, the resin layer 6 may instead be formed of a single cylindrical body made of resin that has an outer diameter that changes stepwise.

The present invention is not limited to the above-described first to sixth embodiments. Various design changes may be made and the embodiments may be combined as appropriate without departing from the scope of the present invention. For example, the resin layer 6 may be formed by applying a resin material in a molten state to the outer peripheral surface of the core wire 2 and curing the resin material. In this case, when the proximal end of the inner coil body 9 is to be connected to the distal end of the resin layer 6 along the axial direction of the core wire 2, the proximal end of the inner coil body 9 may be inserted into the resin layer 6 and be set to the embedded state in the curing process. In the third to sixth embodiments, the proximal end of the inner coil body 9 is connected to the distal end of the resin layer 6 with the second fixing portion 7 interposed therebetween from the viewpoint of productivity. However, the proximal end of the inner coil body 9 may instead be connected to the distal end of the resin layer 6 by, for example, bringing the proximal end of the inner coil body 9 into contact with the distal end of the resin layer 6 without providing the second fixing portion 7 therebetween. In addition, in the third to sixth embodiments, the outer diameter of the inner coil body 9 is preferably less than or equal to the maximum outer diameter of the resin layer 6. In such a case, when the guidewires 1C to 1F are deformed into a curved shape, the inner coil body 9, which is on the distal-end side of the resin layer 6, can be prevented from interfering with the coil body 3. As a result, the distal end portions of the guidewires 1C to 1F exhibit sufficient flexibility. In addition, in the fourth to sixth embodiments, the inner coil body 9 may be omitted.

## Claims

1. A guidewire (1A, 1B, 1C, 1D, 1E, 1F) comprising:
a core wire (2);
a coil body (3) that covers at least a distal end portion of the core wire (2);
a tip portion (4) that fixes a distal end of the core wire (2) and a distal end of the coil body (3) to each other; and
a resin layer (6) formed at least inside the coil body (3) and on an outer peripheral surface of the core wire (2), wherein an outer peripheral surface of the resin layer (6) and an inner peripheral surface of the coil body (3) face each other with a gap therebetween so that the resin layer (6) and the coil body (3) are not in contact with each other.

2. The guidewire according to claim 1, wherein the resin layer (6) is formed on the outer peripheral surface of the core wire (2) so as to extend from a proximal end of the tip portion (4) toward a proximal end of the core wire (2).

3. The guidewire according to claim 1 or 2, further comprising:
an inner coil body (9) that is disposed inside the coil body (3) and covers the core wire (2),
wherein the resin layer (6) is formed at least inside the inner coil body (9) and the outer peripheral surface of the resin layer (6) and an inner peripheral surface of the inner coil body (9) face each other with a gap therebetween so that the resin layer (6) and the inner coil body (9) are not in contact with each other.

4. The guidewire according to claim 1, further comprising:
an inner coil body (9) that is disposed inside the coil body (3) and covers the core wire (2), the resin layer (6) being disposed on a proximal-end side of the inner coil body (9),
wherein a distal end of the inner coil body (9) is fixed to the tip portion (4) and a proximal end of the inner coil body (9) is connected to a distal end of the resin layer (6).

5. The guidewire according to claim 1, further comprising:
an inner coil body (9) that is disposed inside the coil body (3) and covers the core wire (2), the resin layer (6) being disposed on a proximal-end side of the inner coil body (9),
wherein a distal end of the inner coil body (9) is separated from a proximal end of the tip portion (4) toward a proximal end of the guidewire and a proximal end of the inner coil body (9) is connected to a distal end of the resin layer (6).

6. The guidewire according to one of claims 1 to 5, wherein the resin layer (6) is formed of a cylindrical body made of resin.

7. The guidewire according to one of claims 1 to 6, wherein the resin layer (6) has an outer diameter that increases toward a proximal end of the core wire (2).

8. The guidewire according to claim 6, wherein the cylindrical body that forms the resin layer (6) has an outer diameter that increases stepwise toward the proximal end of the core wire (2).
